# EUROPÄISCHE PATENTANMELDUNG

(11) **EP 0 567 205 A1**
(43) Veröffentlichungstag der Anmeldung: **27.10.1993**
(21) Anmeldenummer: 93250110.9
(22) Anmeldetag: 05.09.1987
(51) Int. Cl.: A61K 31/485

(54) **Verwendung von Opiat-Antagonisten zur Herstellung von Mitteln zur Diagnose und Therapie von hypothalamischen Funktionsstörungen**

(30) Priorität: 12.09.1986 DE 3631088
(62) Teilanmeldung aus: 87113006.8
(71) Anmelder: Ferring Arzneimittel GmbH, D-24109 Kiel (DE)
(72) Erfinder: Leyendecker, G., Prof. Dr. med., W-6100 Darmstadt (DE); Wildt, Ludwig, Prof. Dr., W-5300 Bonn 1 (DE)
(74) Vertreter: UEXKÜLL & STOLBERG

(57) **Zusammenfassung**

Die Erfindung betrifft die Verwendung des Opiat-Antagonisten Naltrexon mit der Formel 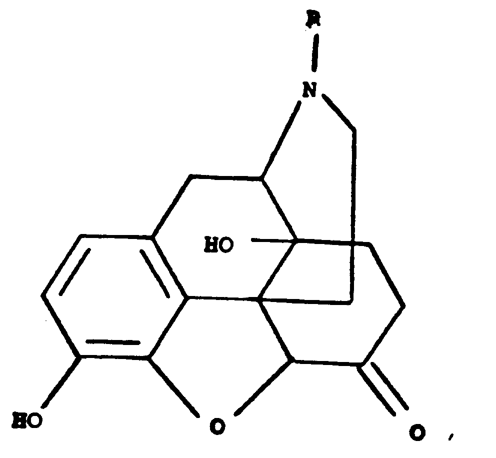
worin R für eine Cyclopropylmethylgruppe steht, zur Herstellung von Arzneimitteln für die Diagnose und Therapie von hypothalamischen Funktionsstörungen, insbesondere hypothalamischen Gonaden-Insuffizenzen.

## Beschreibung

Die Erfindung betrifft die Bereitstellung von Mitteln, Arzneimitteln und Diagnostika, nämlich von Opiat-Antagonisten, zur Therapie und/oder Diagnose von hypothalamischen Funktionsstörungen, insbesondere von hypothalamischen Gonaden-Insuffizienzen, die mit einer erhöhten endogenen Opiataktivität einhergehen.

Im Hypothalamus werden Releasing-Factors gebildet, die steuernd auf die Bildung wichtiger Hormone im Hypophysen-Vorderlappen wirken. Funktionsstörungen des Hypothalamus, die beispielsweise durch Endorphine hervorgerufen werden, führen somit zu einer Störung der Hormonbildung im Hypophysen-Vorderlappen, wodurch wichtige Körperfunktionen wie beispielsweise die Funktion peripherer endokriner Drüsen gestört wird.

So wird beispielsweise der ovarielle Zyklus durch ein Kontrollsystem gesteuert, welches aus dem Hypothalamus, der Hypothyse und dem Ovar besteht. Während des normalen Zyklus wird Gonadotropin-Releasing-Hormon (GnRH) vom Hypothalamus in Form von Pulsen mit einer Frequenz von 60 bis 90 Minuten abgegeben. Hypothalamische Funktionsstörungen, insbesondere hypothalamische Gonaden-Insuffizienzen beruhen auf einem völligen Mangel oder einer Einschränkung der hypothalamischen Sekretion von Gonadotropin-Releasing-Hormon (GnRH), sowie auf einem fehlenden pulsatilen Verhalten dieser Hormone im Serum. Dieser Mangel kann traumatisch bedingt sein; er kann Folge sein eines primären Defektes hypothalamischer Neurone oder kann auf einer verstärkten endogenen Opiataktivität beruhen.
Beispielsweise wird bei der hypothalamischen Amenorrhoe die pulsatielle Freisetzung von GnRH reduziert. Es ist davon auszugehen, daß sowohl freie Frequenz als auch Amplitude der GnRH Sekretion dabei beeinträchtigt sind. Die reduzierte Sekretion von GnRH hat eine ebenfalls reduzierte Sekretion von luteinisierendem Hormon (LH) und follikelstimulierendem Hormon (FSH) an der Hypophyse zur Folge. Daher werden die Ovarien nur ungenügend stimuliert. Das Follikelwachstum kommt entweder nicht zustande oder systiert bevor die volle präovulatorische Reife des Follikels erreicht ist.

Die bisherigen therapeutischen Ansätze beruhten auf einer Substitution der Hormone, die bei Funktionsstörungen des Hypothalamus nur in reduzierter Weise gebildet wurden. Im Falle der vorstehend geschilderten hypothalamischen Amenorrhoe beispielsweise wurde dies durch Injektion von LH und FSH, die aus Urin isoliert wurden, vorgenommen. Der Nachteil einer Hormonbehandlung lag in der Gefahr der überstimulation, da die Verabreichung der Hormone, beispielsweise durch Injektion, nicht genau kontrollierbar bzw. dosierbar war. Eine überstimulation beim Beispiel der Amenorrhoe konnte beispielsweise zu einem exzessiven Follikelwachstum führen.

Einen weiteren Schritt stellte die Subsitutionsbehandlung der Releasing Factors bzw. Releasing Hormone dar, die jedoch eine relativ aufwendige Behandlung erforderte. Im Falle der hypothalamischen Amenorrhoe erfolgte beispielsweise eine Substitutionsbehandlung mit GnRH, bei der durch pulsatielle Infusion des Releasing Hormons die Funktion des Hypothalamus nachgeahmt wurde und dadurch eine Normalisierung der hypophysär-ovariellen Achse erreicht wurde. Hierzu war die Patientin gezwungen, über mehrere Wochen, unter Umständen sogar über Monate, mit einer Infusionspumpe zu leben.

Aufabe der Erfindung ist daher die Bereitstellung eines Mittels zur Therapie und Diagnose von hypothalamischen Funktionsstörungen, insbesondere von hypothalamischen Gonaden-Insuffizienzen, die mit einer erhöhten Opiataktivität einhergehen, das die vorstehenden Nachteile nicht aufweist.

Im Rahmen der Erfindung hat es sich gezeigt, daß der Opiat-Antagonist Naltrexon als derartiges Mittelgeeignet ist. Es wurde gefunden, daß die Verabreichung des Opiat-Antagonisten Naltrexon bei hypothalamischen Funktionsstörungen bzw. hypothalamischen Suppressionen zu einer Normalisierung der Funktion des Hypothalamus führt. Es hat sich gezeigt, daß bei Verabreichung des Opiat-Antagonisten Naltrexon bei hypothalamischen Funktionsstörungen, die zu einer Verminderung bestimmter Hypophysen-Vorderlappenhormone im Blutspiegel von Patienten führten, der Hormongehalt im Blut wieder normalisiert werden konnte.

Insbesondere hat es sich gezeigt, daß der Einsatz des Opiat-Antagonisten Naltrexon zur Therapie und Diagnose von hypothalamischen Funktionsstörungen, besonders hypothalamischen Gonaden-Insuffizienzen, die auf einer verstärkten endogenen Opiataktivität beruhen, wirksam ist.
Der Einsatz des Opiat-Antagonisten Naltrexon kann somit einerseits zur Therapie hypothalamischer Funktionsstörungen verwendet werden, andererseits aber auch zur Diagnose derartiger Störungen, da bei einer Normalisierung des Hormonspiegels im Blut eines Patienten nach Verabreichung des Opiat-Antagonisten auf eine hypothalamische Funktionsstörung geschlossen werden kann.

Die Erfindung betrifft daher Mittel, die den Opiat-Antagonisten Naltrexon enthalten, und zur Diagnose und Therapie von hypothalamischen Funktionsstörungen geeignet sind. Derartige Mittel sind besonders geeignet zur Diagnose und Therapie von hypothalamischen Funktionsstörungen bzw. Suppressionen, die durch Endorphine bedingt werden. Bevorzugt lassen sich den erflndungsgemäßenOpiat-Antagonisten enthaltende Mittel zur Diagnose und Therapie von hypothalamischen Gonaden-Insuffizienzen und besonders von hypothalamischer Amenorrhoe sowie von Gelbkörperschwäche, anovulatorische Blutungen und Oligomenorrhoe verwenden.

Gegenstand der Erfindung ist somit die Verwendung des Opiat-Antagonisten Naltrexon zur Herstellung von Mitteln zur Diagnose und Therapie von hypothalamischen Funktionsstörungen. Bevorzugt ist die Verwendung von Opiat-Antagonisten zur Herstellung von Mitteln zur Diagnose und Therapie von hypothalamischen Gonaden-Insuffizienzen, die mit einer erhöhten endogenen Opiataktivität einhergehen, insbesondere von hypothalamischer Amenorrhoe.

Der erfindungsgemäße Opiat-Antagonist weist die Formel 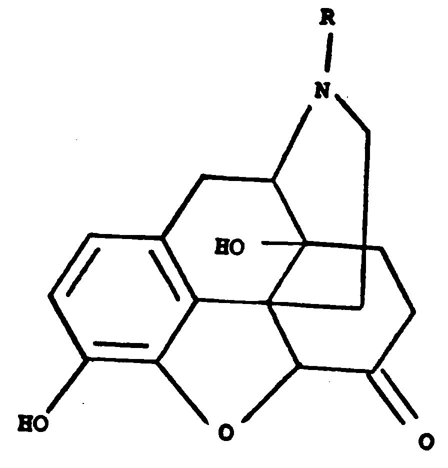
auf, worin R für eine Cyclopropylmethylgruppe steht.

Derartige 4,5-Epoxy-3,14-dihydroxymorphinan-6-on-Derivate und ihre Herstellung werden in der GB-PS 939 287 und der US-PS 3 332 950 beschrieben. Sie wurden bisher als Antagonisten für Narkotika bzw. Opiate eingesetzt. Ihr Hauptverwendungszweck lag in der postoperativen Verabreichung nach der Verwendung von Opiat-Analgetika. Beispielsweise wurden derartige Opiat-Analgetika an Säuglinge unmittelbar nach der Geburt verabreicht, wenn die Mutter vorher Opiate zur Schmerzstillung erhalten hatte.

Die erfindungsgemäß hergestellten Mittel können sowohl zur Therapie als auch zur Diagnose der hypothalamischen Funktionsstörungen eingesetzt werden. Während die eigentliche Therapie erst abgeschlossen ist, wenn unter Gabe des Opiat-Antagonisten ein normaler Zustand, z.B. ein normaler menstrueller Zyklus und evtl. eine Schwangerschaft auf- bzw. eingetreten sind, wird bei Einsatz des Opiat-Antagonisten als Diagnostium lediglich überprüft, ob es unter kurzfristiger Gabe zu einer Anhebung der Serumspiegel von LH und FSH und/oder zum Auftreten eines normalen pulsatilen Musters der interessierenden Hormone im Serum kommt. Diese kurzfristige Zufuhr stellt selbst noch keine Therapie dar, sondern weist bei pos. Testausfall darauf hin, daß der länger dauernde therapeutische Einsatz des Opiat-Antagonisten sinnvoll ist.
In der Praxis wird bei der Verwendung des erfindungsgemäß eingesetzten Opiat-Antagonisten als Diagnostikum bespielsweise wie folgt vorgegangen. Im Blut eines an einer hormonell bedingten Funktionsstörung leidenden Patienten wird der verringerte Hormongehalt bzw. die reduzierte Pulsation eines Hormons bestimmt. Anschließend wird das Opiat verabreicht. Wenn sich daraufhin eine Normalisierung des Hormonspiegels bzw. der Pulsation ergibt, läßt dies den Rückschluß auf eine hypothalamische Suppression als Grund für die verminderte Hormonerzeugung zu. Eine entsprechende Behandlung kann dann erfolgen.

Das 17-(Cyclopropylmethyl)-4,5-epoxy-3,14-dihydroxymorphinan-6-on (Naltrexon)ist zur Diagnose hypothalamischer Funktionsstörungen geeignet. Es hat sich jedoch gezeigt, daß Naltrexon besonders gut zur Therapie hypothalamischer Funktionsstörungen geeignet ist.

Im Rahmen der vorliegenden Erfindung wurden Untersuchungen an Patientinnen mit hypothalamischer Amenorrhoe durchgeführt. Diese Untersuchungen haben überraschenderweise gezeigt, daß durch die Gabe des Opiat-Antagonisten Naltrexon eine Zunahme von Frequenz und Amplitude der LH und FSH Sekretion erreicht wird. Es kann daher davon ausgegangen werden, daß die endogene GnRH Sekretion durch den Opiat-Antagonisten vermehrt wird.

Naltrexon stellt einen Opiat-Antagonisten dar, der oral verabreicht werden kann und eine lange Verweildauer im Plasma aufweist. Die Gabe von Naltrexon an Patientinnen mit hypothalamischer Amenorrhoe führte zur Normalisierung der Gonadotropin-Sekretion, zur Ovulation und zur Bildung eines Corpus luteum.

Die erfindungsgemäß eingesetzten Opiat-Antagonisten enthaltenden Mittel lassen sich, wie die vorstehenden Versuche zeigen, besonders günstig zur Diagnose und Therapie der gestörten Sekretion von GnRH und Gonadotropin bei Patientinnen mit hypothalamischer Amenorrhoe verwenden. Es wird eine Normalisierung des menstruellen Zyklus bewirkt. Es ist daher möglich, durch die Gabe des Opiat-Antagonisten Naltrexon die hypothalamische Amenorrhoe zu behandeln, bzw. bei unklarer Diagnose die Diagnose hypothalamischer Amenorrhoe aus der Reaktion auf den Opiat-Antagonisten zu stellen.

Die vorstehenden Untersuchungen haben gezeigt, daß besonders Naltrexon, ein oral verabreichbarer Opiat-Antagonist, zur Normalisierung des menstruellen Zyklus geeignet ist.

Untersuchungen haben gezeigt, daß die Verabreichung von Naltrexon bei Patientinnen mit normalem Zyklus keinen Einfluß auf die Sekretion der gonadotropen Hormone und auf den Ablauf des Zyklus hat. Es ergibt sich daher die Möglichkeit, den Opiat-Antagonisten zur Diagnose und Therapie hypothalamischer Funktionsstörungen einzusetzen, insbesondere bei solchen, bei denen zu vermuten ist, daß eine Hemmung der entsprechenden Partialfunktion durch vermehrt gebildete Endorphine verursacht ist.

Der erfindungsgemäße Opiat-Antagonist kann oral und/oder parenteral verabreicht werden. Der Verabreichungweg der bekannten einzusetzenden Mittel ist dem Fachmann geläufig. Zur Verabreichung wird der Opiat-Antagonist mit üblichen Hilfs- und Trägerstoffen für die orale und/oder parenterale Verabreichung formuliert. Die Verabreichungsdauer und die zu verabreichenden Mengen des erfindungsgemäß eingesetzten Opiat-Antagonisten richten sich nach dem Ausmaß der zu therapierenden Funktionsstörung. Beispielsweise kann bei streßinduzierten Funktionsstörungen eine einmalige oder kurzzeitig mehrfach weiderholte Verabreichung zum Erfolg führen. Nicht streßbedingte Störungen können eine Langzeittherapie erfordern. So kann z.B. Naltrexon zur Behandlung von hypothalamischer Amenorrhoe für die Dauer eines oder mehreren Menstruationszyklen verabreicht werden. Tägliche Dosen von Naltrexon liegen dabei in der Größenordnung von 50 mg.

Die Verabreichung des Opiat-Antagonisten Naltrexon kann verwendet werden um bei Patientinnen mit Kinderwunsch und hypothalamischer Amenorrhoe die Ovarialfunktion zu normalisieren und das Eintreten einer Schwangerschaft zu ermöglichen. Darüberhinaus ist es außerdem, insbesondere durch die Gabe von Naltrexon, möglich, die gestörte Ovarialfunktion zum Beispiel bei Patientinnen, die eine Amenorrhoe aufgrund einer Streßsituation entwickeln zu normalisieren, wobei die durch die normalerweise eingesetzen Steroidhormone bedingte Problematik vermieden wird. Die hypothalamische Amenorrhoe stellt jedoch einen Extremfall einer hypothalamischen Funktionsstörung dar. Die erfindungsgemäßen Mittel mit Naltrexon können jedoch auch zur Behandlung leichterer Formen von Zyklusstörungen, zum Beispiel Gelbkörperinsuffizienz oder anovulatorische Zyklen, die auf einer leichten Verminderung der hypothalamischen GnRH Sekretion beruhen, verwendet werden. Durch die Bereitstellung der erfindungsgemäßen Mittel wird somit ein wichtiges therapeutisches Problem gelöst. Die Corpus luteum Insuffizienz stellt nämlich heute ein großes therpeutisches Problem dar, da sehr viele Patientinnen mit Kinderwunsch an einer solchen Funktionsstörung leiden und die Therapie der Corpus luteum Insuffizienz mit den bisher zur Verfügung stehenden Mitteln mehr oder weniger unbefriedigend war.

### Beispiel

Die basale Gonadotropin Sekretion von Patientinnen, die an hypothalamischer Amenorrhoe litten, wurde durch Bestimmung von LH und FSH, Prolactin und Ovarien-Steroiden in ßlutproben bewertet, die alle zehn Minuten während 24 Stunden entnommen wurden. Naltrexon wurde dann in einer Dosis von 50 mg pro Tag während 24 oder 28 Tagen verabreicht. 5 Tage und 14 Tage nach dem Beginn der Naltrexonverabreichung wurden wiederholte Blutproben entnommen. An den übrigen Tagen wurden täglich Blutproben entnommen und es wurde eine Ultraschalluntersuchung nach der Naltrexonverabreichung sowie einige Tage danach durchgeführt. Es wurden folgende Ergebnisse ermittelt:

Auf die Verabreichung von Naltrexon folgte eine Zunahme der Frequenz und der Amplitude der pulsatielen Gonadotropin Sekretion sowie eine Zunahme der mittleren LH und FSH Serumkonzentration sowie der Entwicklung der Gonaden-Steroid Sekretion und der Follikel, was durch Ultraschall festgestellt wurde. Eine von 3 Patientinnen ovulierte. Sämtliche Patientinnen menstruierten nach Absetzen des Mittels.

## Patentansprüche

1. Verwendung des Opiat-Antagonisten Naltrexon mit der Formel 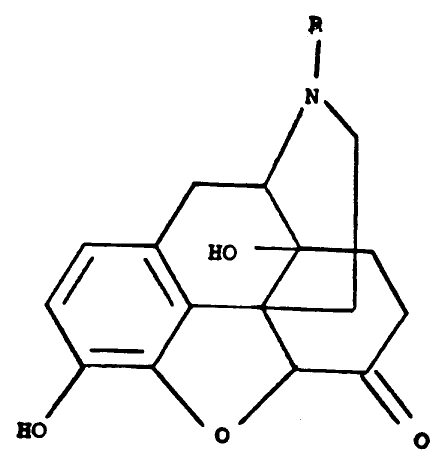 worin R für eine Cyclopropylmethylgruppe steht, zur Herstellung von Arzneimitteln für die Diagnose und Therapie von hypothalamischen Funktionsstörungen, insbesondere hypothalamischen Gonaden-Insuffizenzen.
